Europäisches Patentamt

European Patent Office

Office européen des brevets

(19)

(11) Veröffentlichungsnummer : **0 169 591**
**B1**

(12) **EUROPÄISCHE PATENTSCHRIFT**

(45) Veröffentlichungstag der Patentschrift :
20.09.89

(51) Int. Cl.⁴ : **A 61 B   6/02**, G 05 B   19/42

(21) Anmeldenummer : 85200959.6

(22) Anmeldetag : 18.06.85

(54) Schichtaufnahmegerät.

(30) Priorität : 22.06.84 DE 3423001

(43) Veröffentlichungstag der Anmeldung :
29.01.86 Patentblatt 86/05

(45) Bekanntmachung des Hinweises auf die Patenterteilung : 20.09.89 Patentblatt 89/38

(84) Benannte Vertragsstaaten :
DE FR GB IT NL

(56) Entgegenhaltungen :
DE--A-- 1 936 915
DE--A-- 2 003 407
DE--A-- 2 154 235
DE--A-- 2 746 630
FR--A-- 2 225 911
FR--A-- 2 340 077
US--A-- 3 951 271

(73) Patentinhaber : **Philips Patentverwaltung GmbH**
**Wendenstrasse 35 Postfach 10 51 49**
**D-2000 Hamburg 1 (DE)**
**DE**
**N.V. Philips' Gloeilampenfabrieken**
**Groenewoudseweg 1**
**NL-5621 BA Eindhoven (NL)**
**FR GB IT NL**

(72) Erfinder : **Kunert, Heinz-Peter**
**Amselweg 4**
**D-2081 Tangstedt (DE)**

(74) Vertreter : **Hartmann, Heinrich, Dipl.-Ing. et al**
**Philips Patentverwaltung GmbH Wendenstrasse 35**
**Postfach 10 51 49**
**D-2000 Hamburg 1 (DE)**

EP 0 169 591 B1

Jouve, 18, rue St-Denis, 75001 Paris, France

## Beschreibung

Die Erfindung betrifft ein Schichtaufnahmegerät mit zwei Antrieben zur Verschiebung von Röntgenstrahler und Bildaufnehmer in zwei Richtungen mit einem Speicher, in dem die verschiedenen Verwischungsfiguren zugeordneten Sätze von Antriebssollwerten gespeichert sind, von denen jeweils einer bei der Ausführung einer Schichtaufnahme abrufbar ist.

Aus der DE-AS 19 36 915 ist bereits ein Schichtaufnahmegerät mit zwei Antrieben zur Verschiebung von Röntgenstrahler und Bildaufnehmer in zwei zueinander senkrechten Richtungen bekannt. Weiterhin ist es aus der DE-AS 21 54 235 bekannt, für ein derartiges Schichtaufnahmegerät einen Speicher vorzusehen, in dem die verschiedenen Verwischungsfiguren zugeordneten Sätze von Antriebssollwerten gespeichert sind, von denen jeweils einer bei der Ausführung einer Schichtaufnahme abrufbar ist.

Der Vorteil von Schichtaufnahmegeräten mit zwei voneinander unabhängigen Antrieben gegenüber Schichtaufnahmegeräten mit nur einem Antrieb besteht darin, daß damit grundsätzlich jeder beliebige Typ von Verwischungsfiguren ausgeführt werden kann. In der Praxis können diese Vorteile jedoch nicht ausgenutzt werden, und deshalb können auch mit einem Gerät der eingangs genannten Art nur relativ wenig Verwischungsfiguren ausgeführt werden. Um eine dieser Verwischungsfiguren ausführen zu lassen, betätigt der Benutzer eine zugeordnete Taste, wonach aus dem Speicher ein Satz von Antriebssollwerten aufgerufen wird, der die jeweilige Verwischungsfigur festlegt.

Der Erfindung liegt die Aufgabe zugrunde, ein Schichtaufnahmegerät der eingangs genannten Art so auszubilden, daß der Benutzer selbst Verwischungsfiguren vorgeben bzw. vorhandene Verwischungsfiguren ändern kann.

Diese Aufgabe wird erfindungsgemäß dadurch gelöst, daß der Strahler bzw. der Bildaufnehmer unabhängig von den gespeicherten Antriebssollwerten positionierbar ist, daß ein Istwertgeber zum Erfassen der jeweiligen Position von Strahler bzw. Bildaufnehmer vorgesehen ist, daß ein Rechner zur Bildung der Antriebssollwerte aus den gemessenen Positionswerten vorgesehen ist, und daß die vom Rechner gebildeten Werte in den als Schreib-Lese-Speicher ausgebildeten Speicher übernehmbar sind.

Der Benutzer positioniert also — manuell oder mittels Motorantrieb — den Strahler bzw. den Bildaufnehmer auf einen Bahnpunkt, den der Strahler bzw. der Bildaufnehmer durchlaufen soll. Der Rechner bildet aus den in diesen Positionen gemessenen Istwerten einen Satz von Antriebssollwerten, der in den Schreib-Lese-Speicher übernommen wird. Aus diesem können sie zu einem späteren Zeitpunkt wieder abgerufen werden, wodurch die Antriebe so gesteuert werden, daß die Bahn von Röntgenstrahler und Bildaufnehmer den vom Benutzer vorgegebenen Verlauf hat.

Eine Weiterbildung der Erfindung sieht vor, daß der Rechner aus einem vorgegebenen Verwischungsfigurentyp und einer oder wenigen Positionen des Strahlers einen Satz von Antriebssollwerten für eine entsprechend der Positionsvorgabe verlaufende Verwischungsfigur des vorgegebenen Typs bildet. Dabei wird der gewünschte Typ der Verwischungsfigur, z. B. Kreis, Ellipse, Spirale oder Linearbewegung, vom Benutzer vorgewählt (am Steuerpult des Schichtaufnahmegerätes). Danach werden durch Positionierung des Strahlers die Parameter der gewählten Figur eingestellt. Bei einem Kreis ist dies beispielsweise der Radius, der der Entfernung des Strahlers bzw. des Bildaufnehmers aus der Mittelstellung entspricht. Bei einer anderen Verwischungsfigur muß der Benutzer mehrere Parameter vorgeben; bei einer Ellipse beispielsweise die Länge der großen und der kleinen Halbachse. Der Rechner bildet aus den so vorgegebenen Parametern einen Satz von Antriebssollwerten, die zu einem späteren Zeitpunkt wieder aufrufbar sind. — Hierbei ist also der Figurentyp vorgegeben und der Benutzer kann lediglich einzelne Parameter dieser Verwischungsfigur beeinflussen.

Eine andere Weiterbildung der Erfindung sieht demgegenüber vor, daß der Rechner die Verwischungsfiguren aus einer Vielzahl von Positionswerten bildet. Diese Weiterbildung gestattet es dem Benutzer, vollständig neue Verwischungsfiguren einzustellen. Hierbei bewegt der Benutzer den strahler bzw. den Bildaufnehmer also auf einer Bahn, die diese Teile später bei der Vorgabe dieser Verwischungsfigur ausführen sollen und diese Bahn wird gespeichert, indem die den einzelnen Bahnpunkten zugeordneten Istwerte gespeichert werden. Der Rechner verbindet die Bahnpunkt zu einer stetigen Verwischungsfigur.

Die Erfindung wird nachstehend anhand der Zeichnungen näher erläutert. Es zeigen

Fig. 1 den mechanischen Aufbau eines Schichtaufnahmegerätes, bei dem die Erfindung anwendbar ist,

Fig. 2 ein Prinzipschaltbild eines solchen Gerätes.

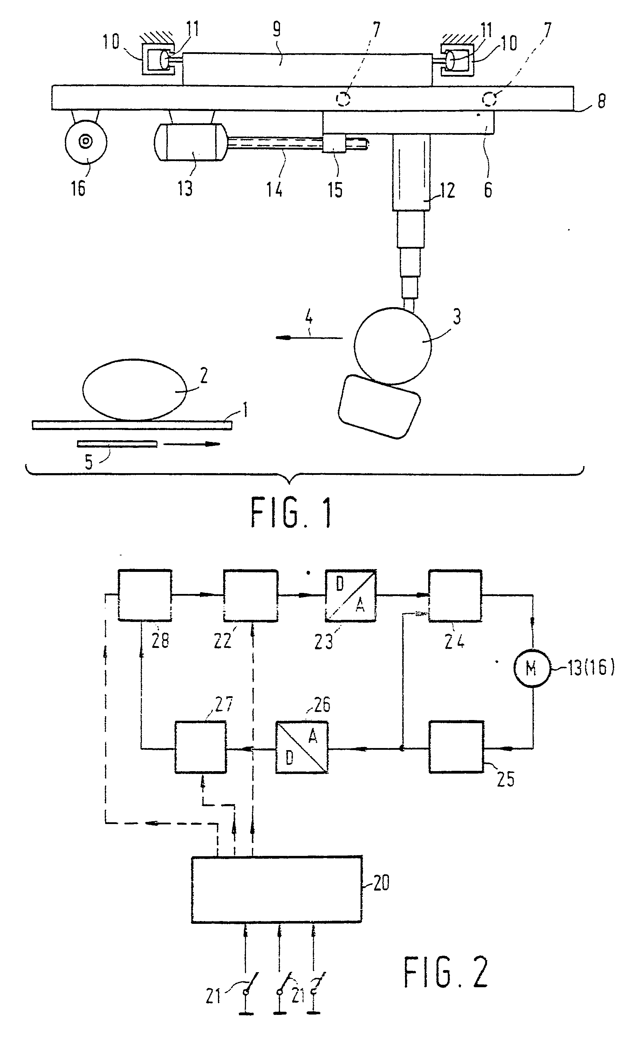

Fig. 1 zeigt die Schmalseite einer Tischplatte 1 eines Schichtaufnahmegerätes, auf der das zu untersuchende Objekt 2 liegt. Das Objekt 2 wird von einem Röntgenstrahler 3 durchstrahlt, der in Richtung des Pfeiles 4 sowie senkrecht zur Zeichenebene bewegbar ist, wobei der Zentralstrahl des Röntgenstrahlers stets auf den gleichen Punkt des Objektes ausgerichtet bleibt. Der Röntgenstrahler ist mit der unter der Tischplatte 1 befindlichen Filmkassette 5 über eine nicht näher dargestellte Lenkstange derart gekuppelt, daß Röntgenstrahler und Filmkassette 5 gegensinnig zueinander bewegt werden.

Der Röntgenstrahler 3 wird von einem teleskopartigen Deckenstativ 12 getragen, das an einem Querwagen 6 befestigt ist, der mittels der Rollen 7 quer zur Richtung der Tischlängsachse in den Schienen 8 verfahrbar ist, die an einem Längswagen 9 befestigt sind, der seinerseits mittels Rollen 11 in an der Decke befestigten Schienen 10 verfahrbar ist, die sich parallel zur Richtung der Längsachse der Tischplatte 1 erstrecken. Am Längswagen 9 ist ein Motor 13 befestigt, der eine Spindel 14 treibt, die in einen am Querwagen 6 befestigten Motor 15 eingreift. Auf diese Weise kann der Querwagen 6 je nach Drehrichtung der Spindel motorisch nach links oder nach rechts verschoben werden. Ein weiterer fest mit dem Längswagen verbundener Motor 16 greift in ähnlicher Weise über eine Spindel in eine Mutter ein (nicht näher dargestellt), die an der Decke befestigt ist, so daß der Längswagen motorisch senkrecht zur Zeichenebene verschoben werden kann.

In Fig. 2 ist ein Blockschaltbild der elektronischen Steuerung eines der Motoren 13 bzw. 16 dargestellt. Eine Steuereinrichtung 20 ruft in einer ersten vom Benutzer durch Betätigen eines der Schalter 21 bestimmten Betriebsart aus einem Speicher 22 einen Satz von Sollwerten ab, die einer bestimmten Verwischungsfigur zugeordnet sind. Diese Sollwerte werden fortlaufend über einen Digital-Analog-Wandler 23 einer den Motor 13 bzw. 16 steuernden Regeleinrichtung 24 zugeführt und mit den von einem Istwertgeber 25 gelieferten Werten verglichen. Der Röntgenstrahler 3 bzw. der Bildaufnehmer 5 durchlaufen dann eine vorgegebene Verwischungsbahn.

Außer dieser Betriebsart ist es möglich, den Strahler bzw. den Bildaufnehmer unabhängig von einer vorgegebenen Verwischungsfigur manuell oder motorisch in eine bestimmte Position zu fahren. Der Istwertgeber 25, der ein dem Abstand des Strahlers aus einer zentrierten Mittenstellung entsprechendes Signal erzeugt und der ein beispielsweise mit der Antriebsspindel des Motors 13 bzw. 16 gekoppelter Positionsgeber sein kann, liefert ein Signal, das von einem Analog-Digital-Wandler 26 in ein digitales Signal umgesetzt und in einem Speicher 27 gespeichert wird. Dieses den jeweiligen Positions-Istwert darstellende Signal wird von einem Rechner 28 weiterverarbeitet, so daß — ggf. nach Verarbeitung einer Vielzahl weiterer Positions-Istwerte — ein Satz von Positions-Sollwerten in den Schreib-Lese-Speicher 22 geliefert wird, der einen neuen Verwischungsfigur zugeordnet ist. Dabei gibt es die Möglichkeit, eine schon bestehende Verwischungsfigur zu ändern oder eine ganz neue Verwischungsfigur zu schaffen.

Wenn lediglich eine bereits gegebene Verwischungsfigur geändert werden soll, d. h. wenn deren Parameter verändert werden sollen, ist es lediglich erforderlich, den Strahler bzw. den Bildaufnehmer auf einen oder wenige Bahnpunkten der geänderten Verwischungsfigur zu positionieren. Soll beispielsweise ein Kreis in einen Kreis mit einem anderen Radius geändert werden, ist es lediglich erforderlich, den Strahler einmal in eine Position zu bringen, deren Abstand dem Radius des neuen Kreises entspricht. Der Rechner ändert dann den im Speicher 22 für die Verwischungsfigur « Kreis » gespeicherten Satz von Sollwerten (durch Multiplikation aller Sollwerte mit einem konstanten Faktor) derart, daß sich bei einem späteren Aufruf dieses Satzes von Sollwerten der Strahler bzw. der Bildaufnehmer einem Kreis durchlaufen, dessen Radius dem Abstand dieser Komponenten von der zentrierten Ruhestellung (in der sich Strahler und Bildaufnehmer auf einer zur Schichtebene senkrechten Geraden befinden) bei der Neuprogrammierung dieser Verwischungsfigur entspricht.

Es ist aber auch möglich, völlig neue Verwischungsfiguren zu schaffen. Der Benutzer muß dann den Strahler bzw. den Bildaufnehmer auf einer Vielzahl von Bahnpunkten der gewünschten Verwischungsfigur positionieren. Aus den dabei gewonnenen im Zwischenspeicher 27 gespeicherten Positions-Istwerten bildet der Rechner 28 einen Satz von Positions-Sollwerten. Wird dieser Satz später abgerufen, dann führt der Strahler bzw. der Bildaufnehmer eine Verwischungsfigur aus, die durch die zuvor vorgegebenen Bahnpunkte verläuft.

## Patentansprüche

1. Schichtaufnahmegerät mit zwei Antrieben zur Verschiebung von Röntgenstrahler und Bildaufnehmer in zwei Richtungen mit einem Speicher, in dem die verschiedenen Verwischungsfiguren zugeordneten Sätze von Antriebssollwerten gespeichert sind, von denen jeweils einer bei der Ausführung einer Schichtaufnahme abrufbar ist, dadurch gekennzeichnet, daß der Strahler bzw. der Bildaufnehmer unabhängig von den gespeicherten Antriebssollwerten positionierbar ist, daß ein Istwertgeber zum Erfassen der jeweiligen Position von Strahler bzw. Bildaufnehmer vorgesehen ist, daß ein Rechner zur Bildung der Antriebssollwerte aus den gemessenen Positionswerten vorgesehen ist, und daß die vom Rechner gebildeten Werte in den als Schreib-Lese-Speicher ausgebildeten Speicher übernehmbar sind.

2. Schichtaufnahmegerät nach Anspruch 1, dadurch gekennzeichnet, daß der Rechner aus einem vorgegebenen Verwischungsfigurentyp und einer oder wenigen Positionen des Strahlers einen Satz von Antriebssollwerten für eine entsprechend der Positionsvorgabe verlaufende Verwischungsfigur des vorgegebenen Typs bildet.

3. Schichtaufnahmegerät nach Anspruch 1, dadurch gekennzeichnet, daß der Rechner die Verwischungsfiguren aus einer Vielzahl von Positionswerten bildet.

## Claims

1. A tomographic apparatus, comprising two drives for displacing an X-ray source and an image detector in two directions, and also comprising a memory for the storage of the sets of drive reference values which are associated with different blurring patterns and one of which can be fetched each time when a sectional radiograph is made, characterized in that the source or the image detector can be positioned independently of the drive reference values stored, there being provided an actual value generator for determining the instantaneous position of the source or the image detector and also an arithmetic device for forming the drive reference values from the measured position values, it being possible to transfer the values calculated by the arithmetic device to the memory which is constructed as a read/write memory.

2. A tomographic apparatus as claimed in Claim 1, characterized in that from a predetermined type of blurring pattern and one or a few positions of the source the arithmetic device forms a set of drive reference values for a blurring pattern of the preset type which extends in accordance with the positional preset.

3. A tomographic apparatus as claimed in Claim 1, characterized in that the arithmetic device forms the blurring patterns from a plurality of position values.

**Revendications**

1. Appareil de tomographie comportant deux mécanismes d'entraînement pour déplacer la source de rayons X et le dispositif radiographique dans deux directions et une mémoire dans laquelle sont stockés les jeux de valeurs de consigne d'entraînement associés à diverses figures de balayage, parmi lesquels un jeu peut être appelé chaque fois qu'une tomographie est réalisée, caractérisé en ce que la source de rayonnement ou le dispositif radiographique peut être positionné indépendamment des valeurs de consigne d'entraînement stockées, qu'un générateur de valeurs réelles est prévu pour détecter la position respective de la source de rayonnement ou du dispositif radiographique, qu'un calculateur est prévu pour former les valeurs de consigne d'entraînement à partir des valeurs de position mesurées et que les valeurs formées par le calculateur peuvent être reprises dans la mémoire ayant la forme d'une mémoire vive.

2. Appareil de tomographie suivant la revendication 1, caractérisé en ce que le calculateur forme, à partir d'un type de figure de balayage prédéfini et d'une ou de plusieurs positions de la source de rayonnement, un jeu de valeurs de consigne d'entraînement pour une figure de balayage du type prédéfini correspondant aux données de position prédéfinies.

3. Appareil de tomographie suivant la revendication 1, caractérisé en ce que le calculateur élabore les figures de balayage à partir d'un grand nombre de valeurs de position.

FIG. 1

FIG. 2